# EUROPEAN PATENT SPECIFICATION

(11) **EP 1 788 499 B1**
(45) Date of publication and mention of the grant of the patent: **18.02.2009**
(21) Application number: 05025352.5
(22) Date of filing: 21.11.2005
(51) Int. Cl.: G06F 19/00, A61M 5/14

(54) **Apparatus for automated optimization of treatment plans**
Vorrichtung zur automatisierten Optimierung von Behandlungsplänen
Appareil pour l'optimisation automatisée de plans de traitement

(43) Date of publication of application: 23.05.2007
(73) Proprietor: BrainLAB AG, 85622 Feldkirchen (DE)
(72) Inventor: Hartlep, Andreas, 83629 Weyarn (DE); Rodriguez Ponce, Maria Immaculada Dr., 81669 München (DE)
(74) Representative: Schwabe - Sandmair - Marx

(56) References cited:
- US-A1- 2003 114 751
- US-A1- 2004 138 551
- US-B1- 6 549 803
- MARDOR Y; RAHAV O; ZAUBERMAN Y; LIDAR Z; OCHERASHVILLI A; DANIELS D; ROTH Y; MAIER S E; ORENSTEIN A; RAM Z: "Convection-enhanced drug delivery: Increased efficacy and magnetic resonance image monitoring" CANCER RESEARCH, vol. 65, no. 15, 1 August 2005 (2005-08-01), pages 6858-6863, XP009064747 United States

## Description

The present invention relates to an apparatus for automated optimization of treatment plans, and especially to the optimization of Convection enhanced delivery (CED) Treatment Plans after tumor resection

Currently the target area for intra-cranial infusions is estimated by the neurosurgeon from medical images, such as MR-images.

In cases of CED treatment after tumor resection, the physician takes a "safety-range" of 2 cm around the resection cavity as infusion target and the area of T2 enhancement that is identified with potential edema. These areas are quite likely areas of remaining and/or migrating tumor cells. The physician then tries to target these areas by implementing catheters and infusing a drug according to a treatment protocol, wherein he might be supported by simulation predictions by software.

A system according to the preamble of claim 1 is disclosed in document US 2003/0114751.

It is an object of the present invention to improve treatment plans.

This object is solved by the apparatus according to the independent claims.

The present invention utilizes the knowledge about infusion processes in tissue, especially in brain tissue. Once the target area is defined, a method or software suggests an optimal infusion procedure in terms of the numbers of catheters to be used, the positioning of the respective catheters, the convection or infusion parameters, such as flow rate, catheter radius and/or infusion time, catheter guidelines or any constraint or condition to be taken into account in order to lead to an optimal target covering by a substance to be delivered by the respective catheter(s).

According to the present invention, in a first approximation, a spherical fluid distribution is calculated for each catheter tip, wherein the backflow of a substance or fluid to be delivered by the respective catheter can optionally be take into account as described in the corresponding European Patent Application with the title "Method and Apparatus for infusing substances" filed together with the present application. An algorithm is developed and integrated into a software that is able to calculate and display the positioning of such spheres, such that the outlined target region is covered.

The present invention uses the concept that the distribution of a fluid or substance to be infused into tissue is basically distributed along spheres or cylinders and solves thus the problem of obtaining a good coverage of the area to be treated by one or more spheres or cylinders being constructed or simulated around delivery devices, such as catheters, which spheres or cylinders can have a respective different radius depending on the infusion plan, if for example the delivery of a fluid through a first catheter is started prior to the delivery through a second or subsequent catheter.

Preferably further assumptions can be taken, such as safety margins which might be set so that e.g. the overlap of spheres or cylinders can be determined. Furthermore, it is possible that the diameter of spheres or cylinders can be adjusted manually or by convection parameters like flow rate, infusion time, pressure and catheter radius. Risk structures can be outlined automatically or manually and can be rated. The overlap of fluid distribution and risk structures can be adjusted. The spheres and trajectories can be adjusted according to catheter placement guidelines.

In order to provide an optimal covering of a selected target area, the design of the infusion process is of major importance. The design consists basically on information regarding to factors like: catheter numbers, selection of infusion parameter (flow rate, pressure head, infusion time), catheter type, position of the catheters, and so on. Traditionally the physician establishes the infusion process without direct software support. Known methods like catheter guidelines support physicians for an optimal covering, however, these guidelines are based on general rules that are independent of arbitrary target volume and shape, so that these catheter guidelines have limited applicability for an individually shaped appropriate infusion process. Furthermore, existing methods consisting of simulation of the infusate transport, deliver information about the distribution of the fluid in tissue so that the physician can modify the catheter placement planning when considering simulation predictions. This approach demands input information by the user like flow rate, infusion time, catheter radius and catheter positioning, so the approach does not directly support the user for a better infusion set-up (e.g. by suggesting appropriate places for catheter positions, flow rate, catheter type, ...) but merely delivers information about an existing set-up that can then be refined by the user according to the delivered information.

In contrary, the invention provides extended support for catheter placement. Beyond simulation prediction, the invention provides information about the optimal infusion process design which satisfies user demands. This demands can be the target volume and in addition the number of catheters, catheter type, infusion parameters and any requirement that the user considers important for an optimal infusion. So with the disclosed invention the user obtains global information, not only regarding to the simulation fluid predictions, but on the optimal infusion process itself. Currently there is no application or device, which directly supports the physician in establishing the most adequate infusion process.

It is an object of the present invention to propose a device for preparing and assisting an infusion, in particular optimally positioning at least one catheter, preferably for interstitial infusion, using which infusion may be performed as successfully as possible using as few catheters as possible. In general, the intention is preferably to infuse a maximum amount of fluid with a minimum number of operations or catheter injections, such that minimally invasive operations can reduce side effects as far as possible, and possible treatment hazards for the patient can be largely excluded.

It is another object of the present invention to propose a device for the improved introduction of substances into a body.

This object is solved by the device in accordance with the independent claim.

There is described a method for arranging at least one catheter on a body, in particular on a head, the individual anatomical structure and in particular the tissue structure being determined body-specifically. Furthermore, the position of the interstitial fluid, in particular the position relative to the surrounding tissue or body structures, the amount and the distribution are determined body-specifically. In general, it is advantageous to determine all the parameters which influence how the infused fluid proceeds, such as for example the type or composition of the fluid, the pressure distribution, or other parameters. Imaging methods, such as nuclear magnetic resonance (MRI) methods, computer tomography (CT) methods, ultrasound methods, X-ray methods, SPECT methods, PET methods or other suitable methods can for example be used to determine the above-mentioned data and information. Advantageously, other examinations or measurements can also be performed, in order for example to determine a pressure or the pressure distribution of the interstitial fluid or the composition of said infused fluid. The body-specific or head-specific information obtained in this way is evaluated and, on the basis of this, it is determined how to position one or more catheters as optimally as possible. To this end, one or more suitable positions for catheters can be predetermined, for example in a body co-ordinate system or in a system-specific co-ordinate system, at which positions one or more catheters can be positioned simultaneously or sequentially, to perform an infusion as optimally as possible.

There is also described a method for simulating fluid infusion through a catheter in a body, in particular in the interstice, wherein anatomical data on the structure of the body and/or the tissue are individually and body-specifically determined as described above, and the position, amount, distribution and/or type of interstitial fluid or tissue is determined body-specifically, and wherein it is further assumed that one or more catheters are positioned simultaneously or sequentially at one or more predetermined locations, such that from this information, the course of infusion, in particular the infusion of fluid into the body, can be simulated in order to find suitable positions for attaching one or more catheters or to verify their effectiveness.

Using a simulation, other infusion parameters may also be determined, optimised or verified, such as for example a flow rate, a positive pressure present on the catheter, catheter geometry, or the like.

The simulation procedure is preferably performed on the assumption that the catheters are positioned on the body as described above.

The catheter/s are advantageously moved to the desired position on the body using known navigation methods. To this end, active or passive markers, such as for example reflective surfaces, can be attached to the catheter.

Preferably, parameters influencing the infusion of the fluid, such as for example the flow characteristics of a particular fluid can be determined in a specific type of tissue can be determined and used to determine the optimal position of a catheter or for a simulation. Such body-specific parameters can, for example, be stored in a database and can have been determined by examination, before the method of simulation is performed.

Parameters describing the properties of the fluid to be infused may likewise be provided in a database and can be used for example to plan the arrangement of a catheter or to simulate infusing the fluid. The viscosity, the interaction between a particular fluid and a particular tissue, the flow characteristics in a particular type of tissue, or other information can for example be stored in a database, so as to be able to perform the methods of simulation, on the basis of this.

Preferably, the methods are performed using a database containing information and parameters for one or more different types of catheter available. For example, said database may include data on the geometry, in particular the diameter, of the catheter, the material, the surface and its properties when interacting with tissue or fluids to be drained, wherein one or more catheters can be selected automatically and/or by an operator.

Information on possible, advantageous ways of adapting, changing or processing the catheters which are to be used can advantageously be determined and outputted by the method, in order to arrange at least one catheter on a body. For example, an optimal catheter length can be determined, such that a standardised catheter to be used with the inventive method can be cut to a desired length or modified in some other way.

Preferably, other parameters influencing infusion can be determined, such as a positive pressure to be applied to a catheter, using which the best possible infusion can be obtained. In this way, the infusion rate of the fluid, usually in the range of a few ml/h, can be influenced and regulated.

Advantageously, a verification method can be performed, i.e. for example, further data can be captured intra-operatively, for example using an imaging method or other measurement, in order to determine the body-specific structures changed by the infusion and/or the position, distribution and amount of the fluid after partial or complete infusion. On the one hand, this information can be used to verify a infusion carried out and also, on the other hand, to correct or reposition one or more catheters, as may be necessary, to replace catheters, to change the flow rate or to influence other parameters relevant to infusion, in order for example to take into account, for the further course of the infusion, the fact that an already reduced pressure or infused fluid may cause a change in the position of a body or tissue structure and that the infusion plan may have to be changed, for example by repositioning a catheter or changing parameters.

There is also described a method for controlling infusion, wherein a substance is introduced into a body, for example by means of one or more catheters, at at least one location on said body, and a pressure or negative pressure is applied to the body at at least one other location, in particular at a region of the body or interior of the body, such that the distribution of a substance introduced into the body at at least one location can be influenced by applying a pressure or a negative pressure to other locations on the body. It is thus possible for example to establish preferred flow or spreading directions for a substance introduced, in order to introduce said substance as precisely as possible into particular regions of the body. Furthermore, applying a negative pressure can reduce or remove a possibly damaging internal pressure in the body, which would be further increased by introducing a substance. To apply a negative pressure, catheters can for example be positioned in the interstice or in the ventricles, in order to apply a negative pressure at at least one desired location on the body and to influence for example the distribution or flow direction of a substance introduced into the body.

In general, the method for controlling infusion can be used in combination with one or more of the method steps described above. For example, based on body-specific anatomical and/or tissue structure data, the catheters can be suitably positioned to introduce a substance and/or to apply a positive pressure or a negative pressure, an infusion process can be simulated to determine the distribution of a substance in the body. Furthermore, the catheters can be positioned using navigation methods. It is also possible to use information stored in databases to plan or perform the method as described above.

There is also disclosed a computer program which can be loaded into the memory of a computer and which includes sections of software code, using which one or more steps of the methods described above can be performed when the program is run on a computer. There is further disclosed a computer program product stored on a computer-compatible medium or data carrier and comprising the computer program just described.

According to an aspect, the invention relates to a device for simulating fluid infusion into a body, comprising a data capture device for capturing structural data of the body and/or the position of a fluid in a body, such as for example a nuclear spin tomograph and a computer system for determining the arrangement of at least one catheter on a body, and/or comprising an input device for inputting the position of at least one catheter in order to simulate the fluid infusion from the body, using the body-specific and fluid-related data determined by the data capture device.

In general, with the present invention one or more of the method steps described above can be performed. To this end, for example, databases are provided for storing parameters or characteristics of one or more catheters, particular body parameters, fluid parameters and/or infusion parameters.

In order to perform the infusion, a device for setting the infusion flow rate, such as for example a pump, is advantageously provided, which is able to generate a desired positive in order to be able to perform infusion as planned and/or simulated by the methods described.

Known methods using markers, such as for example the VectorVision^{®} system distributed by the applicant, can be used to navigate one or more catheters, in order to ensure that said catheters are optimally positioned and seated.

With respect to a device and methods for administering a substance, which may be used in combination with the invention, reference is made to European patent application No. 01 128 614.3, filed by the Applicant on November 30, 2001 and corresponding US 2003/0114751 A1.

The invention will now be described by way of preferred embodiments. There is shown:
- Figure 1: a schematic diagram of a method for planning and performing a infusion;
- Figure 2: a simplified flow chart of an infusion; and
- Figure 3: a device which may be used to plan and perform a infusion.

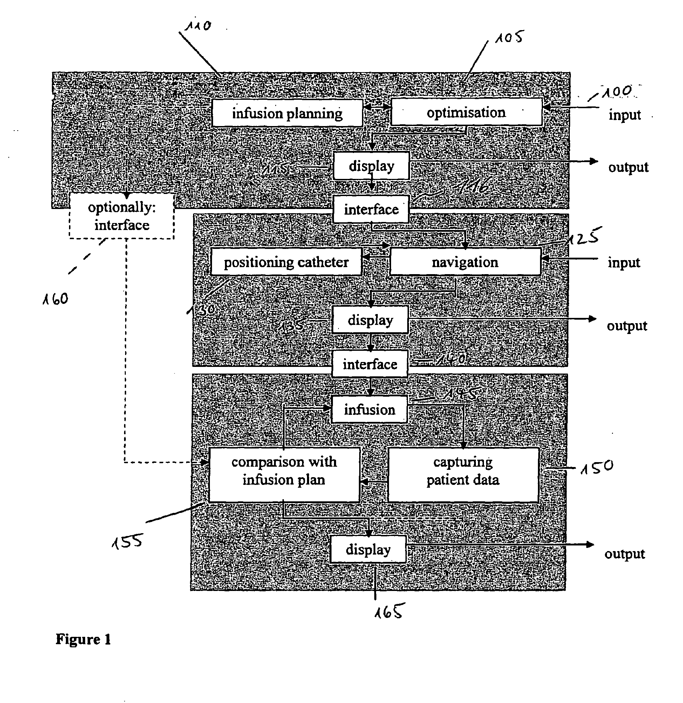

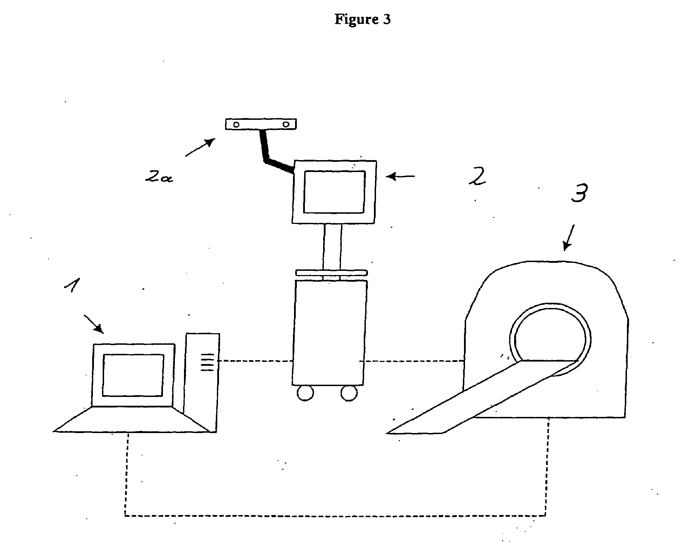

Figure 1 shows a schematic flow chart for preparing and performing an infusion. As shown in Figure 1, patient data are inputted, step 100, for example by a nuclear spin tomograph, which are used to determine one or more particular regions for positioning catheters for infusion, and to plan the infusion to be performed. Said data can be obtained, for example using the nuclear spin resonance system 3 as shown schematically in Figure 3, once a patient to be treated has been examined. Using parameters for the properties of the tissue structures and for various types of catheter, stored for example in databases, one or more catheters suitable for the infusion can be selected, once the exact position of the target area to be treated by infusion and of the body or tissue structure has been determined. The parameters of the body or the patient, obtained for example using the nuclear spin resonance method, can be used together with the catheter parameters and the fluid parameters, also for example stored in databases, to plan the infusion, step 110. In this way, the course of the infusion to be performed can be optimised in step 105 by balancing the conditions of infusing as great a proportion of the fluid as possible into the body structure or the target tissue, while doing so in as few operations as possible. In general, as few catheters or needles should be positioned as possible, said catheters or needles being supplied through as few access points as possible. This optimised planning of the infusion is outputted via a display, step 115, such that for example a two-dimensional or three-dimensional representation can be outputted by imaging various incision planes, in order to display the resultant infusion plan.

The infusion plan produced in this way is transmitted via an interface 116 to a navigation system, step 125, such as for example the VectorVision^{®} system shown schematically in Figure 3, in order to position the selected catheter or catheters at the predetermined locations on the body, step 130, based on the planning data. These can be positioned automatically, for example using a robot, or by hand if guided by the navigation system, wherein a display device can indicate whether a catheter has been positioned correctly or whether it still has to be moved in a particular direction, step 135.

Once the catheter or catheters have been successfully positioned at step 130, the actual infusion is performed at step 145 using the infusion parameters predetermined by the plan, such as for example a flow rate which is constant or which changes with time. To this end, patient data are again captured at step 150 to determine the actual distribution of the fluid in the body or tissue. Using the parameters predetermined by the plan and the infusion simulation results based on them, a comparison is made at step 155 between the actual infusion, in particular the distribution of the partially infused fluid, and the predetermined distribution of fluid, and as appropriate the parameters is altered, such as for example the flow rate, the infusion amount or a pressure or suction applied to the catheter for performing infusion, preferably taking into account known active mechanisms, in order to obtain the desired, planned infusion result. Again, the actual distribution of the interstitial fluid measured can preferably be outputted together at step 165 with any deviations and correction methods via a display, for example to enable an operator to intercede in the infusion method manually.

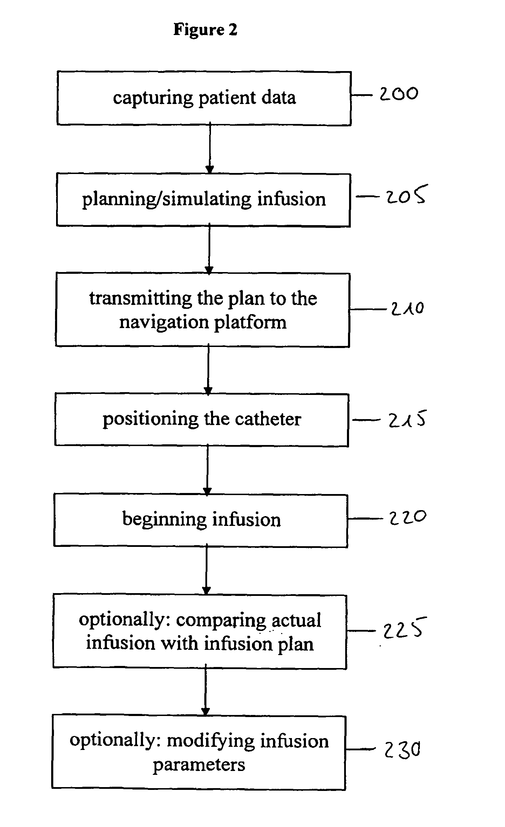

Figure 2 schematically shows a simplified sequence of planning and performing an infusion. Firstly, patient data are captured at step 200 using a diagnostic imaging method, such as for example a nuclear spin resonance method, to obtain the current patient parameters, such as for example tissue density, pressure and position of a fluid to be drained. Using the patient parameters determined in this way, as well as catheter and infusion parameters obtained from a database and/or predetermined for a specific infusion, the infusion is planned and/or simulated at step 205. Based on the parameter data determined in this way, the infusion plan is transmitted at step 210 to a navigation platform, by which the catheter or catheters are to be positioned at step 215 on the patient as provided for in the infusion plan. Infusion begins at step 220 once the catheters have been positioned and is performed using the planned and as appropriate simulated parameters, wherein - as shown in Figures 1 and 2 - a comparison is optionally made between the infusion actually performed and the infusion plan at step 225 and, in the event of deviations, the corresponding parameters are modified at step 230 , preferably by utilising known active mechanisms.

Figure 3 schematically shows a device which may be used to plan and perform a infusion in accordance with the invention. Patient data are obtained in a nuclear spin tomograph 3 and transmitted to a planning system 1 and to a navigation system 2. Using the navigation system 2, the catheter or catheters are positioned at a desired location on the body using, for example, known reflectors or markers attached to one or more of the catheters, positional data of the markers being captured by infrared cameras 2a. The planning system 1 determines the suitable catheter parameters and infusion parameters for a predetermined infusion to be performed, using the patient parameters determined by the nuclear spin resonance system 3, in order to perform the infusion.

## Claims

1. System for adjusting infusion parameters that provide coverage of a selected target area in tissue for direct infusion of the fluid comprising an imaging device (3), a processor (1) connected to the imaging device (3) for obtaining patient-specific medical or anatomical data and a data bank (1) connected to the processor (1) containing information to calculate infusion relevant parameters from the imaging data obtained from the imaging device and an infusion system containing at least one delivery device (3) and an injecting mechanism, preferably a pump, which can be controlled in terms of operating time and/or delivery rate by the computer (1) based on the information obtained from the imaging device (3) and/or the data bank (1) to cover a target volume with a substance delivered or infused by the at least one delivery device, **characterised in that** the coverage of the selected target volume is determined by an algorithm for calculation of optimal packing of spheres or cylinders in a selected volume to calculate a spherical or cylindrical fluid distribution, which spheres or cylinders can have a respective different radius, wherein the diameter of the spheres or cylinders is adjusted.

## Patentansprüche

1. System zum Einstellen von Infusionsparametern, die eine Abdeckung eines ausgewählten Zielbereichs in einem Gewebe für eine direkte Infusion des Fluids ergeben, das umfasst: eine Abbildungsvorrichtung (3), einen Prozessor (1), der mit der Abbildungsvorrichtung (3) verbunden ist, um patientenspezifische medizinische oder anatomische Daten zu erhalten, und eine Datenbank (1), die mit dem Prozessor (1) verbunden ist und Informationen enthält, um für die Infusion relevante Parameter aus den von der Abbildungsvorrichtung erhaltenen Abbildungsdaten zu berechnen, und ein Infusionssystem, das wenigstens eine Abgabevorrichtung (3) und einen Injektionsmechanismus, vorzugsweise eine Pumpe, enthält, deren Betriebsdauer und/oder deren Abgaberate durch den Computer (1) anhand der von der Abbildungsvorrichtung (3) und/oder der Datenbank (1) erhaltenen Informationen gesteuert werden können, um ein Zielvolumen mit einer Substanz abzudecken, die durch die wenigstens eine Abgabevorrichtung abgegeben oder infundiert wird, **dadurch gekennzeichnet, dass** die Abdeckung des ausgewählten Zielvolumens durch einen Algorithmus für die Berechnung einer optimalen Packung von Kugeln oder Zylindern in einem ausgewählten Volumen bestimmt wird, um eine sphärische oder zylindrische Fluidverteilung zu berechnen, wobei die Kugeln oder Zylinder unterschiedliche Radien haben können, wobei der Durchmesser der Kugeln oder der Zylinder eingestellt wird.

## Revendications

1. Système pour ajuster des paramètres d'injection qui assurent la couverture d'une zone cible sélectionnée dans un tissu pour l'injection directe du fluide, comprenant un dispositif d'imagerie (3), un processeur (1) connecté au dispositif d'imagerie (3) pour obtenir des données médicales ou anatomiques spécifiques au patient et une banque de données (1) connectée au processeur (1) contenant une information pour calculer des paramètres appropriés à l'injection à partir des données d'imagerie obtenues à partir du dispositif d'imagerie et un système d'injection contenant au moins un dispositif de délivrance (3) et un mécanisme d'injection, de préférence une pompe, qui peut être commandé vis-à-vis du temps de fonctionnement et/ou du débit de délivrance par l'ordinateur (1) en fonction de l'information obtenue à partir du dispositif d'imagerie (3) et/ou de la banque de données (1) de façon à couvrir un volume cible avec une substance délivrée ou injectée par le dispositif de délivrance au nombre d'au moins un, **caractérisé en ce que** la couverture du volume cible sélectionné est déterminée par un algorithme pour le calcul du remplissage optimal par des sphères ou des cylindres dans un volume sélectionné de façon à calculer une répartition de fluide sphérique ou cylindrique, ces sphères ou ces cylindres pouvant avoir un rayon respectif différent, le diamètre des sphères ou des cylindres étant ajusté.
